Europäisches Patentamt

**European Patent Office**

**Office européen des brevets**

(11) Numéro de publication: **0 387 176**

**A1**

(12) **DEMANDE DE BREVET EUROPEEN**

(21) Numéro de dépôt: **90480037.2**

(22) Date de dépôt: **09.03.90**

(51) Int. Cl.5 **A61N 1/32**

(30) Priorité: **10.03.89 FR 8903276**

(43) Date de publication de la demande:
**12.09.90 Bulletin 90/37**

(84) Etats contractants désignés:
**AT BE CH DE DK ES FR GB GR IT LI LU NL SE**

(71) Demandeur: **Gerbal, Pierre**
**Château d'Azur, 45, avenue de la Source**
**F-06100 Gairaut(FR)**

(72) Inventeur: **Gerbal, Pierre**
**Château d'Azur, 45, avenue de la Source**
**F-06100 Gairaut(FR)**

(74) Mandataire: **Hautier, Jean-Louis**
**Cabinet Hautier Office Méditerranéen de**
**Brevets d'Invention et de Marques 24 rue**
**Masséna**
**F-06000 Nice(FR)**

(54) **Appareil pour le traitement des rides utilisant des ondes électriques unidirectionnelles de quelques micro-ampères et de fréquence basse et des produits cosmétiques ionisables.**

(57) L'invention a pour objet un appareil pour le traitement des rides.

L'appareil comporte une tête mobile tournante interchangeable (1) ou fixe montée sur un moyen faisant office de rotule et dont la sensibilité de la mobilité est réglable, ladite tête (1) forme une palette (9) dont la tranche ou arête métallique est conductrice et assez fine pour pénêtrer dans les berges entre les deux sillons de la ride, ladite tranche a un fil à électrodes (11) d'un diamètre d'un demi-millimètre séparé par un espace de quelques millimètres.

Traitement des rides notamment du visage et du cou.

Fig. 1

## Appareil pour le traitement des rides utilisant des ondes électriques unidirectionnelles de quelques micro-ampères et de fréquence basse et des produits cosmétiques ionisables.

L'invention a pour objet un appareil pour le traitement des rides utilisant des ondes électriques unidirectionnelles de quelques micro-ampères et de fréquence basse et des produits cosmétiques ionisables tels que des crèmes.

L'état de la technique peut être défini par les brevets suivants :

- FR-A-2.391.738/77.29194 : Appareil permettant de maintenir le processus négatif de cellules humaines, animales et végétales et/ou de faire pénétrer des substances dans les cellules, caractérisé en ce qu'un générateur susceptible d'être influencé au moyen d'un circuit de commande génère des impulsions haute fréquence présentant une fréquence de répétition et une durée réglables et en ce qu'un circuit électrique produit une tension continue invariable et une tension continue pulsatoire pour l'ionisation, ainsi que des impulsions d'une forme déterminée et d'une fréquence de répétition variable pour la faradisation du tissu à traiter.

Ce brevet décrit un appareil qui génère des impulsions de haute fréquence pour l'électrothérapie.

- FR-A-2.545.361/83.07663 : La présente invention concerne un stimulateur électrique ou autres destiné à fournir des impulsions électriques pour des traitements par électrothérapie et notamment pour la stimulation acupuncturale.

L'appareil selon l'invention se caractérise en ce qu'il comporte un générateur d'impulsions de fréquence précise, stable et réglable, un ou plusieurs ensembles électriques et/ou électroniques recevant les impulsions issues dudit générateur et comportant un moyen de sélection et de modulation de la forme des impulsions et/ou du train d'impulsions à la sortie dudit ensemble électrique et/ou électronique, au moins un générateur fournissant un courant indépendant des variations de résistance des points de stimulation et commandé par des impulsions et/ou le train d'impulsions élaborés par le moyen de sélection et de modulation, et au moins un moyen permettant la variation dans le temps de l'intensité du courant à la sortie du générateur de courant auquel il est associé.

Ce brevet décrit un appareil spécifique pour le traitement par la stimulation acupuncturale.

- FR-A-2.535.974/83.18120 : Appareil de traitement de tissus vivants par stimulation au moyen d'impulsions de courant électrique et/ou d'ondes électromagnétiques. Un dispositif de commande et d'affichage est relié à un générateur d'impulsions à basse fréquence de courant continu, ainsi qu'à un générateur de courant alternatif à haute fréquence et à un émetteur laser. Les générateurs et l'émetteur sont reliés à une sonde endonasale commune par l'intermédiaire d'un commutateur commandé par le dispositif de commande et d'affichage. La sonde permet d'appliquer successivement ou simultanément les impulsions de courant électrique émises par les générateurs et le rayon laser provenant de l'émetteur en une même région de la muqueuse endonasale d'un patient. Application à titre d'appareil de traitement thérapeutique, notamment pour le rétablissement de l'équilibre neurovégétatif, et l'obtention d'une action anti-inflammatoire et reconstituante. L'appareil est spécifique pour être utilisé avec une sonde endonasale.

- FR-A-2.563.437 : L'appareil est remarquable en ce qu'il présente deux sorties conformées pour autoriser chacune le branchement électrique d'un organe adaptateur, au moins l'un desdits organes étant agencé à son extrémité libre pour l'adaptation d'accessoires divers, en étant conformé intérieurement pour recevoir un élément vibrant et des connexions permettant l'alimentation d'un élément chauffant que présentent lesdits accessoires, ledit adaptateur étant d'autre part agencé à son extrémité en combinaison avec l'accessoire correspondant pour autoriser la transmission de la vibration à l'ensemble ainsi que la conduction du courant jusqu'à l'extrémité de l'accessoire permettant la ionisation ; l'autre organe adaptateur faisant office de poignée de préhension ou de fixation (ventouse), l'ensemble de l'appareil étant asservi à une électronique de commande conformée pour assurer un effet chauffant, un effet vibrateur et un effet ionisant avec des formes quelconques d'ondes de courant.

FR-A-2.087.227 : Activateurs électroniques de pénétration dermique constitués par des générateurs dont l'alimentation peut être ou non, contenue dans le même bloc et ces générateurs étant utilisés avec des électrodes de travail et de formes les plus diverses.

FR-A-2.391.738 : Appareil permettant de maintenir le potentiel négatif de cellules humaines, animales et végétales et/ou de faire pénétrer des substances dans les cellules, caractérisé en ce qu'un générateur susceptible d'être influencé au moyen d'un circuit de commande génère des impulsions haute fréquence présentant une fréquence de répétition et une durée réglables et en ce qu'un circuit électrique produit une tension continue invariable et une tension continue pulsatoire pour l'ionisation ainsi que des impulsions d'une forme déterminée et d'une fréquence de répétition variable pour la faradisation du tissu à traiter.

Ces différents appareils ne permettent pas de traiter de manière efficace les rides car ils ne sont pas conçus de manière spécifique.

L'appareil selon l'invention est spécifique pour le traitement des rides ; il autorise, de par la conception de sa tête, une application directe sur les deux berges du sillon formé par la ride.

A cet effet, l'appareil selon l'invention est du type appareil à main dont l'applicateur permet d'être en contact avec la peau ou d'opérer par balayage, il comprend un générateur d'ondes électriques unidirectionnelles réglable au moyen d'un circuit de commande, ledit circuit est alimenté par des piles ou accumulateur , un convertisseur continu, un filtre associé au convertisseur qui constitue une source de tension, un convertisseur continu alternatif à deux fréquences sélecté par un commutateur ; ledit applicateur est formé d'une tête mobile interchangeable ou fixe montée sur un moyen faisant office de rotule et dont la sensibilité de la mobilité est réglable.

L'appareil est caractérisé par le fait que ladite tête formant une palette dont la tranche ou arête métallique est conductrice et assez fine pour pénétrer dans les berges entre les deux sillons de la ride, ladite tête est solidaire d'un contact qui actionne le générateur d'ondes, dès l'application, et que les ondes ont une intensité de quelques micro-ampères et de fréquence basse. La tranche a un fil à électrodes d'un diamètre d'environ un demi-millimètre séparé par un espace de 2mm ; ladite palette a une forme de secteur angulaire dont les rayons sont issus du boîtier et l'arc fait office d'applicateur.

Procédé de traitement des rides en utilisant l'appareil ci-dessus mentionné et un produit cosmétique conducteur ionisable qui facilite l'ionisation tel qu'une crème ou un gel.

La palette ou embout interchangeable est emmanchée par son axe sur un autre axe monté sensiblement perpendiculairement à la poignée de l'appareil, ledit support traversant une bille orientable maintenue dans son logement-capuchon par un ressort. Une vis assure le réglage de la tension du ressort afin de régler la mobilité de la tête de l'appareil.

Les dessins ci-joints sont donnés à titre d'exemple indicatif et non limitatif. Ils représentent un mode de réalisation préféré selon l'invention. Ils permettront de comprendre aisément l'invention.

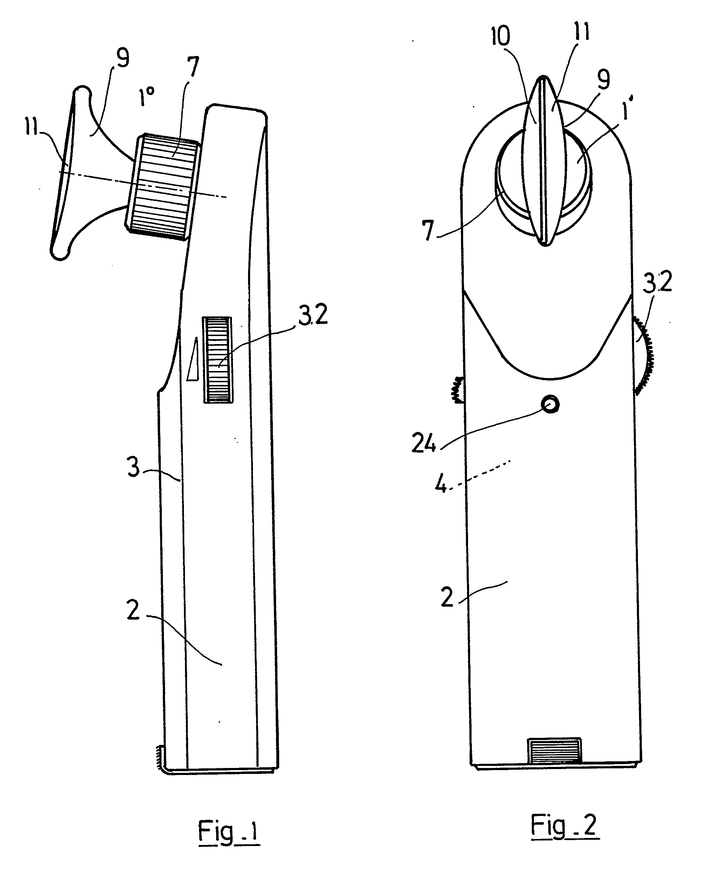

La figure 1 est une vue en perspective vue de côté de l'appareil.

La figure 2 est une vue de face de l'appareil.

La figure 3 est une vue schématique d'une prise polarisée.

La figure 4 est une vue schématique de la tête de l'appareil vue en coupe selon l'axe longitudinal du manche et de la tête.

La figure 5 est une vue schématique vue en coupe de la palette.

La figure 6 est une vue du schéma électrique de l'appareil.

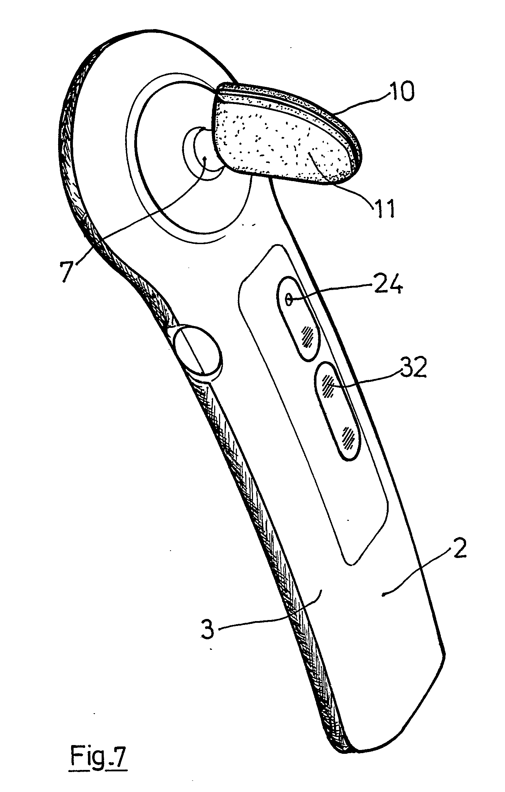

La figure 7 est une vue d'un autre mode de réalisation.

L'appareil est du type appareil à main avec une tête 1 qui fait office d'applicateur et un manche 2 qui fait office de boîtier 3 pour loger notamment le générateur d'ondes électriques 4.

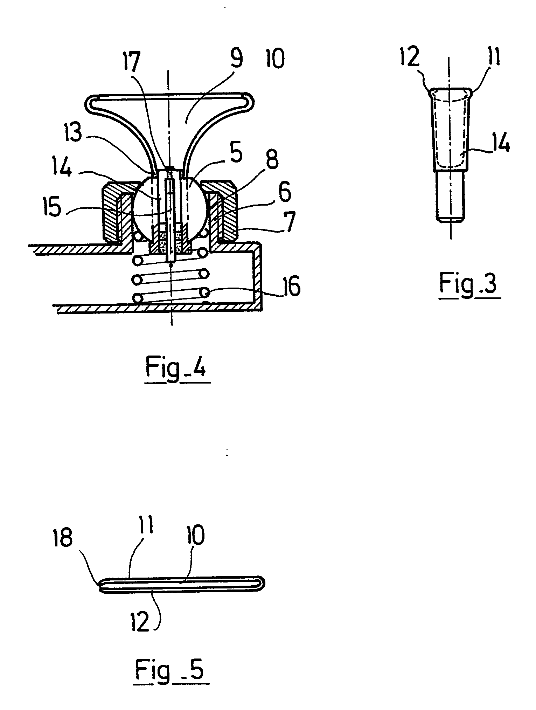

La tête 1 est amovible, mobile sur un moyen faisant office de rotule 5 telle qu'une bille 5 logée dans un logement 6 fermé par un capuchon 7 qui s'emboîte dans une dérivation 8 du corps du manche 2. Cette dérivation 8 est sensiblement perpendiculaire au manche 2. L'extrémité de la tête 1 qui fait office d'applicateur se termine par une palette 9 dont la tranche 10 est conductrice sur chacune de ses faces 11 et 12, voir la figure 5. La tranche 10 se compose d'un fil à électrodes d'un diamètre de 0,6 à 0,7 mm séparé par un espace 18 d'environ 2 mm. Sur la face 11, ou électrode 11, un courant positif circule, sur la face 12, ou électrode 12, un courant négatif circule.

L'axe 13 de cette palette 9 se termine par une prise polarisée 14, voir la figure 3. Cette prise 14 s'enfiche sur un axe 15 qui traverse la bille orientable 5 logée dans le logement 6 fermé par un capuchon 7. La bille orientable 5 qui assure la mobilité de la palette 9 est maintenue dans son logement 6 par un ressort 16 qui traverse le logement 6 pour prendre appui contre les parois du boîtier 3.

Une vis non représentée assure le réglage de la tension du ressort et permet ainsi de régler la sensibilité de mobilité de la tête 1 de l'appareil.

Selon le mode de réalisation représenté à la figure 4, une vis 17 permet de régler la longueur de l'axe 15, ce qui permet de régler la sensibilité de la mobilité de la tête 1 de l'appareil.

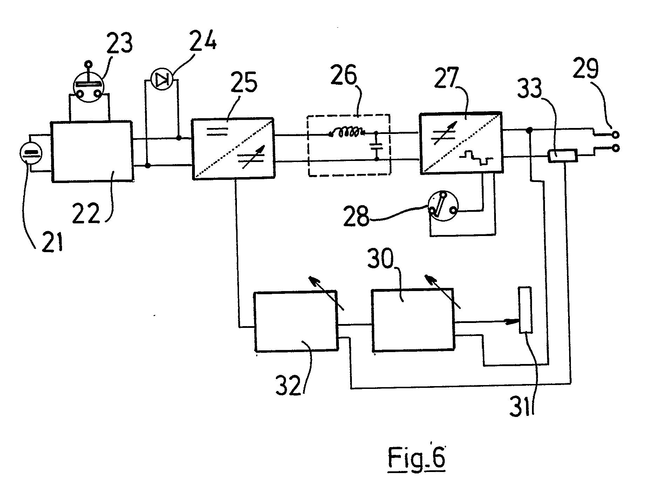

Le schéma représenté à la figure 6 représente les éléments suivants :
- L'appareil est formé du boîtier 3 et de la tête 1. A l'intérieur du boîtier 3 se trouvent la pile 21 et une plaque de circuit électronique où sont montés les parties électroniques ; de ce circuit, partent les fils électriques qui vont jusqu'à la prise 14, lesdits fils électriques alimentent les faces ou électrodes 11 et 12 sur la tête mobile tournante 1.

Les différents éléments électroniques de ce circuit sont les suivants:

21 - Pile 9v, 0,3 A/heure.

22 - Opérateur, à retard de 2mn, activé à la fermeture de 23, l'appareil s'arrête d'émettre des ondes électriques toutes les deux minutes.

23 - Contacteur monté sur la tête d'électrodes actionné lors de l'application.

24 - Photodiode indiquant le fonctionnement de l'appareil par un voyant lumineux ou éventuellement bip sonore.

25 - Convertisseur continu, continu variable 9v/90v.

26- Filtre LC associé à 25, pour constituer une source de tension.

27- Convertisseur continu-alternatif à deux fréquences fixes 40 ou 80 Hz sélectées par le commutateur 28.

28 - Commutateur de sélection de fréquence 40 ou 80 Hz.

29 - Electrodes de l'applicateur tension Max = 90v, courant Max = 5,5 mA. (débitant sur une résistance de la peau du visage variant entre 1K... et 17 K...).

30 - Amplificateur de tension pour réguler la tension des électrodes à la valeur consignée par le potentiomètre 31.

31 - Potentiomètre de consigne pour régler la puissance à transférer dans la peau.

32 - Amplificateur d'intensité, limitant le courant dans la peau à 5,5 mA Max.

33 - Shunt pour la référence du courant de limitation.

Voir la figure 6.

Le générateur d'ondes électriques unidirectionnelles, dont l'intensité est de quelques micro-ampères et de fréquence basse (0 à 400 Hz) agit par électrostimulation du tissu conjonctif sous-jacent à la ride, et exerce une régénération des berges de la ride en une dizaine de séances. Chaque stimulation dure environ 2 minutes. La séance globale étant de 15 minutes environ. Il n'y a aucune contre-indication ni allergie. Il est possible d'obtenir un traitement du vieillissement cutané avec effet de lissage médical en combinant la stimulation des points d'acupuncture du visage et du cou. L'application se fait par pose directe de la tête de l'appareil sur les berges de la ride ou par un mouvement de balayage sur une zone plus étendue.

Il est préférable d'augmenter l'intensité du courant jusqu'à sensation d'un léger picotement de la zone d'application. Selon l'invention il faut utiliser, en combinaison, des produits cosmétiques ionisables pour en augmenter l'efficacité. Les zones traitées par l'appareil sont notamment : le visage, le cou et le décolleté.

La peau vieillie et ridée présente un épiderme aminci. La couche basale ne produit plus suffisamment de cellules nouvelles. Les fibrilles de la couche de Malpighi se désagrègent. Les tissus s'affaissent et présentent un aspect fripé. S'il est relativement facile de ralentir et de stopper parfois, la multiplication des rides, l'appareil selon l'invention peut rendre au revêtement cutané un aspect plus plaisant et plus frais.

Un traitement anti-rides doit viser à :

A/ la réhydratation des tissus cutanés ;

B/ la stimulation de la couche basale de l'épiderme ;

C/ la régénération de l'élastine et du collagène, ce qui est le plus difficile à l'heure actuelle.

Les rides sont des plissures cutanées apparaissant par les modifications biologiques des tissus.

## LES COURANTS UTILISES PAR L'APPAREIL

- Ils n'ont pas d'effet chauffant.

- Ils produisent une contraction physiologique des muscles et agissent, par conséquent, sans brutalité et sans provoquer de sensation désagréable suivie de phénomènes de vasoconstriction.

- Ils ne donnent pas lieu à des phénomènes d'intolérance.

- Ils ne pénètrent pas la voûte crânienne et les os.

- Ils ne provoquent aucun trouble pouvant retentir sur l'état général du fait que les électrodes sont conçues pour maintenir la densité du courant à un niveau où tout risque de pénétration est écarté.

Les soins esthétiques du visage effectués au moyen de l'appareil selon l'invention sont pratiqués avec des électrodes de petites dimensions, faciles à manier et à nettoyer, conçues d'habitude pour que l'intensité du courant soit maintenue à un faible niveau, par exemple de quelques micro-ampères et de fréquence basse (0 à 400 Hz).

## LES PRINCIPALES INDICATIONS DE CES COURANTS OU ONDES ELECTRIQUES DANS LA PRATIQUE DES SOINS ESTHETIQUES

1/ Ils ont un effet correspondant à ceux des manoeuvres faciales habituelles (effeurages, vibrations, pétrissages). Ils agissent sur le tissu conjonctif et le tissu musculaire.

2/ Ils exercent une action tonique sur les fibres élastiques du derme, contribuant ainsi à combattre la flaccidité cutanée.

3/ Ils assurent une meilleure répartition des échanges cellulaires au niveau de la peau.

4/ Ils occupent une place importante dans l'ensemble des soins mis en oeuvre pour la plastique du visage, la beauté du corps et l'esthétique de la ligne.

L'effet du courant conjugue trois facteurs :

a) - Hypervascularisation tissulaire

b) - Résorbtion des oedèmes

c) - Augmentation de la production de collagène et d'élastine.

On note une modification de la polarité cellulaire.

En effet, lors du vieillissement, il apparaitrait un état de dépolarisation des membranes cellulaires.

Le courant électrique adéquat appliqué à un tissu biologique modifie l'équilibre des charges électriques de part et d'autre des membranes cellulaires.

L'originalité de l'appareil et de son procédé de mise en oeuvre réside dans le fait de l'application de la tête 1 directement sur les deux berges du sillon formé par la ride qui induit une stimulation électrique sur une zone précise à traiter.

L'impulsion électrique va permettre une repolarisation avec la possibilité de diélectolyse ou ionophorèse permettant une pénétration cutanée de produits cosmétiques ionisables existant sous forme de liquide ou de gel. L'action des substances organiques cosmétiques ionisées se concentre aussi dans les zones superficielles de la peau, où elles agissent au maximum sans que l'on ait à craindre une action générale.

## REFERENCES

1. tête
2. manche
3. boîtier
4. générateur d'ondes électriques
5. rotule ou bille
6. logement
7. capuchon
8. dérivation
9. palette
10. tranche de la palette
11. face ou électrode
12. face ou électrode
13. axe de la palette
14. prise polarisée
15. axe
16. ressort
17. vis
18. espace
21. pile
22. opérateur à retard
23. contacteur
24. photodiode
25. convertisseur continu
26. filtre
27. convertisseur continu-alternatif
28. commutateur de sélection
29. électrodes de l'applicateur
30. amplificateur de tension
31. potentiomètre
32. amplificateur d'intensité
33. shunt

## Revendications

1. Appareil pour le traitement des rides utilisant des ondes électriques unidirectionnelles de quelques micro-ampères et de fréquence basse du type appareil à main composé d'une tête formant l'applicateur et d'un manche contenant un boîtier, ledit boîtier contenant un générateur électrique d'ondes unidirectionnelles, réglable au moyen d'un circuit de commande, ledit circuit est alimenté par des piles, ledit circuit comprenant un convertisseur continu, un filtre associé au convertisseur pour constituer une source de tension, un convertisseur continu alternatif sélecté par le commutateur ; de ce circuit partent des fils électriques qui vont jusqu'aux électrodes de la tête; ledit appareil comporte une tête mobile tournante interchangeable (1) ou fixe montée sur un moyen faisant office de rotule (5) et dont la sensibilité de la mobilité est réglable caractérisé par le fait

que ladite tête (1) forme une palette (9) dont la tranche ou arête métallique (10) est conductrice et assez fine pour pénétrer dans les berges entre les deux sillons de la ride, ladite tranche (10) a un fil à électrodes (11, 12) d'un diamètre d'un demi-millimètre séparé par un espace (18) de quelques millimètres.

2. Procédé de traitement des rides utilisant l'appareil selon la revendication 1, caractérisé par le fait

que préalablement au passage de l'appareil dans les rides, celles-ci ont reçu des produits cosmétiques ionisables sous forme de liquide ou de gel.

3. Appareil selon la revendication 1, caractérisé par le fait

que la palette (9) a une forme de secteur angulaire dont les rayons sont issus du boîtier (3) et l'arc fait office d'applicateur.

4. Appareil selon la revendication 1, caractérisé par le fait

que le moyen faisant office de rotule (5) est une bille (5) logée dans un logement (6) fermé par un capuchon (7) qui s'emboîte dans une dérivation (8) du corps du manche (2) ; cette dérivation (8) est sensiblement perpendiculaire au manche (2).

5. Appareil selon l'une quelconque des revendications 1 ou 4, caractérisé par le fait

que l'axe (13) de cette palette (9) se termine par une prise polarisée (14) ; cette prise (14) s'enfiche sur un axe (15) qui traverse la bille orientable (5) logée dans le logement (6) fermé par un capuchon (7); la bille orientable (5) qui assure la mobilité de la palette (9) est maintenue dans son logement (6) par un ressort (16) qui traverse le logement (6) pour prendre appui contre les parois du boîtier (3).

6. Appareil selon la revendication 5, caractérisé par le fait

qu'une vis assure le réglage de la tension du ressort et permet ainsi de régler la sensibilité de mobilité de la tête (1) de l'appareil.

7. Appareil selon la revendication 5, caractérisé par le fait

qu'une vis (17) permet de régler la longueur de l'axe (15), ce qui permet de régler la sensibilité de la mobilité de la tête (1) de l'appareil.

8. Appareil selon la revendication 1, caractérisé par le fait

que le circuit électronique comporte un opérateur à retard (32) qui arrête l'émission des ondes électriques toutes les deux ou trois minutes.

9. Appareil selon la revendication 1, caractérisé par le fait

que la tête (1) de l'électrode est pourvue d'un contacteur qui actionne le générateur lors de l'application.

10. Appareil selon la revendication 1, caractérisé par le fait

qu'il comporte une photodiode indiquant par un signal lumineux le fonctionnement de l'appareil ; ledit fonctionnement peut également être indiqué par un signal sonore.

Fig. 1

Fig. 2

EP 0 387 176 A1

Fig.4

Fig.3

Fig.5

Fig.6

Fig.7

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | EP-A-143748 (MASTADO) <br> * le document en entier * | 1 | A61N1/32 |
| A | CH-A-313679 (BÄCHLER-BÄHLER) <br> * page 1, lignes 5 - 12 * <br> * page 1, lignes 19 - 40 * <br> * page 2, lignes 44 - 66 * <br> * page 3, lignes 3 - 7 * | 1, 4-6 | |
| A | GB-A-244901 (BAKER) <br> * page 2, ligne 103 - page 3, ligne 37 * | 1, 3-5 | |
| D,A | FR-A-2563437 (COCHET) <br> * page 4, lignes 15 - 18 * <br> * page 6, ligne 1 - page 7, ligne 31 * | 1-3 | |
| D,A | FR-A-2087227 (LE CLERC) <br> * page 1, lignes 12 - 19 * <br> * page 1, ligne 32 - page 2, ligne 8 * <br> * page 5, ligne 36 - page 6, ligne 4 * | 1-3, 10 | |
| A | DE-C-538579 (ERBACHER) <br> * le document en entier * | 1, 9 | **DOMAINES TECHNIQUES RECHERCHES (Int. Cl.5 )** <br><br> A61N |
| D,A | FR-A-2391738 (CARBA) <br> * page 8, ligne 33 - page 9, ligne 11 * | 1, 8 | |

Le présent rapport a été établi pour toutes les revendications

| Lieu de la recherche | Date d'achèvement de la recherche | Examinateur |
|---|---|---|
| LA HAYE | 18 JUIN 1990 | LEMERCIER D.L.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)